# EUROPÄISCHE PATENTANMELDUNG

(11) **EP 0 586 848 A2**
(43) Veröffentlichungstag der Anmeldung: **16.03.1994**
(21) Anmeldenummer: 93111829.3
(22) Anmeldetag: 24.07.1993
(51) Int. Cl.: A61L 27/00, A61F 2/36

(54) **Implantat**

(30) Priorität: 10.09.1992 DE 4230339
(71) Anmelder: MAN Ceramics GmbH, 94453 Deggendorf (DE)
(72) Erfinder: Siebels, Wolfgang, Dipl.-Ing., D-94469 Deggendorf (DE); Scheer, Wolfgang, Dr. rer. nat. Dipl.-Ing., D-83607 Holzkirchen (DE)

(57) **Zusammenfassung**

Zur Herstellung von Implantaten wird eine Materialwahl vorgeschlagen, die dem Implantat optimierte tribologische Eigenschaften verleiht, die insbesondere in einem geringen Verschleiß bestehen. Die Verwendung von Kohlenstoffasern aus Pechprecursor in Verbindung mit einer Matrix aus Duroplast bietet eine Kombination, die dem Implantat gute Gleiteigenschaften mit geringem Verschleiß verleiht, wobei der Verbundwerkstoff gleichzeitig eine ausreichende Druckfestigkeit aufweist.

## Beschreibung

Die Erfindung bezieht sich auf ein Implantat aus biokompatiblem Material, das auf Festigkeitseigenschaften und auf tribologische Eigenschaften optimiert ist.

Endoprothesen, wie z. B. Gelenk-, Bandscheibenimplantate werden in der Regel so ausgelegt, daß sie den mechanischen Beanspruchungen standhalten. Das Hauptoptimierungskriterium ist dabei die Festigkeit des Implantats.

Aus DÖRRE, E.; RICHTER, H. G. ; WILLMANN, G.: Bruchlast keramischer Kugelköpfe Von Hüftgelenkprothesen. In: Biomedizinische Technik 36, 1991, S. 305 - 307, ist es bekannt, daß bei Reibungen ausgesetzten Implantaten, wie Gelenkimplantaten, eine hohe Festigkeit alleine oft keine dauerhafte Funktion der Prothese gewährleistet, sondern daß zusätzlich eine Optimierung der tribologischen Eigenschaften notwendig ist; eine Lösung dazu ist nicht gegeben.

Der Erfindung liegt die Aufgabe zugrunde, Implantate der eingangs genannten Art zu entwickeln, die eine hohe Lebensdauer im implantierten Zustand haben.

Die Aufgabe wird erfindungsgemäß mit den Merkmalen des Anspruchs 1 gelöst.

Durch entsprechende Wahl, nicht nur des Materials der Faser, sondern auch durch deren Kristallstruktur, deren Einbringung in die Matrix, Länge, mechanische Eigenschaften, Volumenanteil im Bauteil, kann die Optimierung der tribologischen Eigenschaften in Verbindung mit einem entsprechenden Gegenpart in einem wesentlich höheren Maße als bisher optimiert werden. Die spezifische Auslegung richtet sich jeweils nach dem mit der Reibfläche zusammenwirkenden Gegenbauteil.

Es ist zwar bekannt, daß die Gleiteigenschaften von Materialien durch Füllstoffe, z. B. Fasern, verbessert werden können, aber es werden dazu keine Kriterien angegeben. Zum Beispiel ist es aus der DE bekannt, daß eine Gelenkkugel mit einer Gelenkpfanne aus C-faserverstärktem Werkstoff optimierte Gleiteigenschaften hat. Wie aus der Zeitschrift Biomedizinische Technik, Band 34, Heft 12/1989, S. 316, entnehmbar ist, gibt es aber mehrere C-Fasertypen mit stark differierenden Eigenschaften.

Der Erfindung liegt nämlich die Erkenntnis zugrunde, daß die tribologischen Eigenschaften nicht nur durch das Füll- bzw. Fasermaterial alleine, sondern auch von der Faserstruktur, dem Faseranteil, Geometrie, Einbettung in die Matrix und von den mechanischen Eigenschaften der Fasern beeinflußt werden.

Implantate sind aufgrund der ständigen Bewegung des Patienten einem hohen Reibungsverschleiß ausgesetzt, weshalb eine Auslegung der Fasern vorzugsweise schwerpunktmäßig auf geringen Verschleiß gerichtet sein kann.

Gemäß einer Ausgestaltung der Erfindung werden Fasern mit unterschiedlichen Querschnitten verwendet. Gut eignen sich Fasern mit einem mittleren Durchmesser von etwa 13 µm, wobei die Durchmesser zwischen 5 bis 20 µm variieren.

Für sehr harte Gegenparts, wie z. B. aus Stahl und insbesondere Keramik, sollte die Zugfestigkeit der Fasern relativ niedrig sein, d. h. unterhalb von 1.500 MPa. Damit leisten die Fasern für den obigen Reibpartner einen höheren Beitrag zur Gleitfähigkeit. Der E-Modul der Fasern soll vorzugsweise zwischen 150 bis 190 GPa, insbesondere 170 GPa auf Zug liegen.

Ein weiterer Beitrag zur Verbesserung der Verschleißfestigkeit im Zusammenhang mit einem harten Gegenpart wird dadurch erreicht, daß die Fasern in regelloser Orientierung in die Matrix eingebettet sind und daß Fasern ohne Sizing verwendet werden. Die Fasern sind dabei nicht mit einem Fertigungshilfsstoff umgeben, der eine Zwischenschicht zur Matrix darstellt und negative biologische Reaktionen hervorrufen könnte.

Es hat sich herausgestellt, daß Fasern besonders gut geeignet sind, deren Kohlenstoffanteil nicht unter 97 Gewichts-% zu liegen kommen und deren Wasserstoffanteil 0,7 Gew.-% nicht übersteigen. Fasern aus etwa 98,6 Gew.-% Kohlenstoffanteil und 0,5 Gew.-% Wasserstoffanteil führen zu einem Implantat, das ausreichende Festigkeit, hohe Gleitfähigkeit und sehr geringen verschleiß hat.

Bei einem Faseranteil im Implantatvolumen größer als 30% ist die Bedeutung des Verbundsystems auf die Fasern zurückführbar, es wird deshalb bevorzugt ein Volumenanteil über 30% für die Fasern genommen.

Bei Verwendung eines Matrixmaterials mit einer Druckfestigkeit oberhalb von 100 MPa ergibt sich eine Kombination mit guten Verschleiß-, Festigkeits- und Gleitwerten, wenn der Faseranteil im Faser-Matrix-Gesamtvolumen etwa 40 bis 50% einnimmt. Damit erhält das Implantat eine hohe Druckfestigkeit und gute Korrosionseigenschaften im Körpermilieu.

Eine optimale Kombination besteht in der Verwendung von aus Pechprecursor hergestellten Kohlenstoffasern mit einem Duroplast, insbesondere Polyphenylglicidylether. Es hat sich herausgestellt, daß dieses Verbundmaterial eine 10-fach höhere Druckfestigkeit hat als Polyethylen (PE), das aufgrund der guten Gleiteigenschaften für Pfannen von Gelenkimplantaten verwendet wird. Dieses bekannte Implantatmaterial hat aber ungenügende Druck- und Reibfestigkeiten und schlechte Fließeigenschaften mit der Folge, daß körperunverträgliche Abriebpartikel Funktionsstörungen hervorrufen. Außerdem sind derartige Implantate nicht dampfsterilisierbar. Gebräuchlich sind ferner Endlosfasern aus PAN (Polyacrylnitril), die einen exakten bzw. gleichmäßigen Durchmesser haben. Implantate mit der erfindungsgemäßen Materialkombination zeigen gegenüber solchen aus den bekannten Fasern deutlich verbesserte Eigenschaften.

Ein weiterer Beitrag zur Verbesserung der tribologischen Eigenschaften wird mit Fasern, insbesondere C-Fasern mit einer Kristallstruktur, die lamellenartige Schichten in radialer Richtung aufweist, erreicht. Derartige Fasern erlauben die Herstellung von sehr glatten Reibflächen und unterstützen somit die Verschleißfestigkeit. Durch die Kristallstruktur wird ein Abgleiten von Faseroberflächenschichten parallel zur Faserlängsachse begünstigt.

Bei einer Prothese nach der Erfindung treten nicht nur die Nachteile von bekannten Lösungen nicht auf, sondern sie hat noch weitere Vorteile zu verzeichnen. Die im wesentlich geringeren Umfang auftretenden Abriebpartikel sind gut körperverträglich. Das Anwachsverhalten ist sehr gut, hier wirkt sich die bessere elektrische Leitfähigkeit günstig aus. Außerdem entspricht der Elastizitätsmodul bei dem Verbund-C-Fasern/Polyphenylglicidylether dem von Knochen, damit sind keine Metallarmierungen wie beim PE notwendig. Dieses hat die weiteren positiven Wirkungen, daß das Implantat röntgendurchlässig bleibt und keine Störungen bei den Diagnoseverfahren Computertomografie und Kernspintomografie verursacht.

Für Implantate zum Ersatz von Knochen, wie z. B. Gelenkimplantate, Bandscheibenersatz und dergleichen, wird demzufolge ein Material oder eine Kombination von mehreren Materialien verwendet, die dem Endprodukt optimale tribologische Eigenschaften verleiht, wobei insbesondere ein geringer Verschleiß und darüber hinaus gute Gleiteigenschaften neben einer hohen Festigkeit zu verzeichnen sind.

Gemäß einer Ausführungsform werden Kohlenstoffasern mit unterschiedlichen Durchmessern aus Pechprecursor verwendet, deren Elementarfäden unverklebt sind. Die Elementarfäden haben zur Bildung einer Durchmesserpalette unterschiedliche Einzeldurchmesser. Die Fasern werden in Verbindung mit einem Duroplast im bekannten Preßverfahren zum gewünschten Implantat geformt, wobei die Fasern in regelloser Orientierung in der Matrix eingebettet sind.

Nach der Pressung werden die Fasern in Verbindung mit der Matrix durch Schleifen und Polieren an die Oberfläche gebracht, wo sie zur Gleitfunktion beitragen.

Die gemäß vorstehend ausgelegten Fasern eignen sich insbesondere für die Kombination mit einem Gleitpartner, bei dem der Gegenpart aus einem sehr harten Werkstoff besteht, wie Stahl, Titan, Keramik, aber insbesondere hochdichte Aluminiumoxid-Keramik.

Die Erfindung erstreckt sich auf ein als Gelenkpfanne ausgebildetes Implantat, das die Merkmale des Anspruchs 8 aufweist.

Hierbei handelt es sich um eine einteilige Pfanne, die im wesentlichen aus einem Material besteht, das unter den biokompatiblen Materialien lediglich aufgrund seiner tribologischen Eigenschaften gewählt wird, d.h. minimalen Verschleiß hat. Zur Unterstützung des knöchernen Anwachsens ist die Pfanne mit einem Flechtwerk überzogen (Makroporosität), das mit der Pfanne im Klebverband innig verbunden ist, das außerdem einen Beitrag zur mechanischen Festigkeit leistet. Das Flechtwerk wird vorzugsweise mit geringem Harz- oder Matrixmaterialanteil gefertigt, um eine hohe Oberflächenrauhigkeit zu erhalten (Mikroporosität).

Die erfindungsgemäße einteilige Pfanne hat gegenüber den bekannten zwei- oder mehrteiligen Gelenkpfannen den Vorteil, daß sie keine Verbindungsprobleme aufweist. Bei den bekannten mehrteiligen Gelenkpfannen (wie sie beispielsweise in DE 29 03 366 beschrieben sind) ist eine aus zwei Halb-Hohlkugeln bestehende Gelenkpfanne vorgesehen, die formschlüssig ineinander gesteckt die Gelenkpfanne bildet. Die innere mit einem Kugelkopf zusammenwirkende Halbschale wird dabei nach tribologischen Eigenschaften ausgelegt, während die übrigen Eigenschaften durch die äußere Halbkugel eingebracht werden. Diese Trennung bedingt jedoch besondere Maßnahmen für die sichere Zusammenfügung der beiden Halbschalen.

Die erfindungsgemäße Pfanne hat dagegen den Vorteil, daß sie fertigungstechnisch sehr einfach und funktionsmäßig optimal herstellbar ist. Die Pfanne wird als rotationssymmetrisches Bauteil ohne Einkerbungen, Nuten, Rillen, Nasen oder dergleichen gefertigt, d.h., in einem Arbeitsgang ist der Kernteil der Pfanne herstellbar. In einem zweiten Arbeitsgang wird die Pfanne am Außenumfang mittels einer Flechtmaschine mit dem Flechtwerk überzogen. Die Enden der Flechtfasern werden zu einem asymmetrisch angeordneten Zapfen zusammengezogen, was im selben Flechtarbeitsgang gemacht wird. Mit diesen zwei sehr einfachen Arbeitsgängen wird eine Pfanne geschaffen, die in Verbindung mit der Wahl eines verschleißgünstigen Materials für die Pfanne und entsprechenden Fasern, wie z.B. die bewährten Kohlenstoffasern und Matrixmaterialien, eine dauerhafte und gut anwachsbare Prothese bietet, bei der der Zapfen für eine drehsichere Lage der Pfanne sorgt.

Als Material für die Pfanne können duroplastische Matrixwerkstoffe, z.B. Epoxidharze oder Triazinharze oder thermoplastische Matrixwerkstoffe, z.B. Polyetherketone oder Polysulfone jeweils verstärkt mit Fasern, bevorzugt aus Kohlenstoff, verwendet werden.

Da bekannt ist, daß Knochen um den Pfannenrand herumwächst, kann die Kante der Stirnfläche abgeschrägt ausgebildet sein, so daß das Flechtwerk die Pfanne an der Schrägfläche umgreift, aber die Stirnfläche freiläßt. Es ist dafür auch eine Ringschulter oder andere Ringvertiefung geeignet, in die das Flechtwerk zur Sicherung der Verbindung mit der Pfanne hintergreifen kann.

Ein guter Tribopartner für das erfindungsgemäße Implantat ist hochdichte Aluminium-Oxidkeramik.

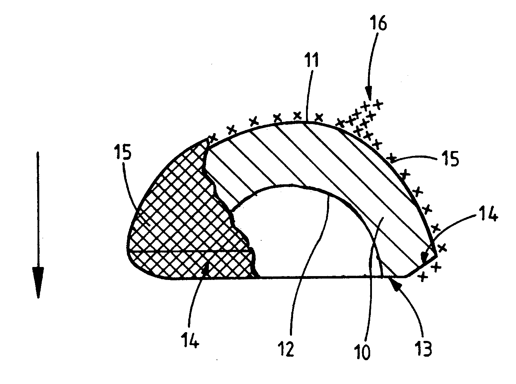

In der Zeichnung ist als Ausführungsbeispiel der Erfindung eine Gelenkpfanne schematisch dargestellt.

Die Gelenkpfanne 10 besteht aus einem homogenen Material, das gute Gleiteigenschaften hat und verschleißfest ist.

Die Pfanne 10 wird einstückig in einem Arbeitsgang mit der äußeren halbkugelförmigen Oberfläche 11 und der inneren halbkugelförmigen Lauffläche 12 aus Faserverbundwerkstoff hergestellt, wobei die Fasern nach tribologischen Gesichtspunkten gewählt und verarbeitet werden. Die Fasereigenschaften richten sich jeweils nach dem Gleitgegenpart, in diesem Fall nach dem Material einer nicht dargestellten Gelenkkugel, die mit der Lauffläche 12 der Pfanne 10 zusammenwirkt.

Für eine Gelenkkugel aus hochdichter Al₂O₃-Keramik wurde eine Pfanne 10 verwendet, die aus kurzen Precursor-Fasern besteht, deren Kohlenstoffanteil 98,6 Gew.-% und Wasserstoffanteil ca. 0,5 Gew.-% betrug. Die 50 - 500 µm langen Fasern hatten eine Kristallstruktur mit radialgerichteten Lamellenschichten und waren um 10 µm dick. Der E-Modul der Fasern betrug 170 GPa.

Diese Kurzfasern wurden in einem Volumenanteil von 50% mit Polyphenglicidylether zur Bildung der Pfanne 10 in einem bekannten Preßverfahren verarbeitet, wobei die Fasern in regelloser Orientierung im Matrixmaterial eingebettet waren. Die Lauffläche 12 wurde anschließend mit Diamantpaste absteigender Körnung geläppt. Aufgrund der Kristallstruktur der Fasern wurde eine hochglatte Lauffläche 12 erreicht.

Bei Dauerversuchen mit der vorbeschriebenen Pfanne und einem Gleitpartner aus Al₂O₃-Kermaik konnte ein ausgezeichnetes Ergebnis nachgewiesen werden, bei dem kein nennenswerter Abrieb festgestellt werden konnte.

Es wurde bei der beschriebenen Auslegung der Fasern nämlich festgestellt, daß die Fasern eine Schmierwirkung ohne Verwendung eines Schmiermittels besitzen, so daß das Implantat auch ohne Körperflüssigkeit im Patienten eine sehr hohe Lebensdauer erreichen kann.

Die Pfanne 10 ist in der Formgebung am Übergang der Stirnseite 13 zur äußeren Oberfläche 11 abgeschrägt. Die dadurch gebildete Ringfläche 14 dient zum Umgreifen eines Flechtwerks 15, das die Außenfläche 11 der Pfanne 10 umgibt. Das aus Fasern aus biokompatiblem Material bestehende Flechtwerk 15 geht durch Anwendung eines Harzes oder anderem Matrixmaterial eine Klebverbindung mit der Pfanne 10 ein, diese Verbindung wird durch das Umgreifen des Flechtwerks 15 um die Ringkante 14 auf Dauer sichergestellt. Durch die Abschrägung 14 bleibt die Ebene der Stirnseite 13 frei von Fasern. Anstelle einer Abschrägung kann eine Ringschulter, Ringnut oder dergleichen vorgesehen werden.

Das Flechtwerk 15 wird in einer einfachen Weise dadurch hergestellt, daß die Pfanne 10 in Pfeilrichtung, d.h. mit der Stirnseite 13 zuerst durch ein nicht dargestelltes Fadenauge einer Flechtmaschine gebracht wird. Die nach dem Überziehen der äußeren Fläche von dieser abhebenden Fasern werden zu einem außermittig angeordneten Zapfen 16 zusammengeklebt. Dieser Zapfen 16 dient bei der implantierten Pfanne 10 als Drehsicherung.

Die strukturierte Oberfläche des Flechtwerks 15 fördert das Einwachsen des Knochens. Durch Verwendung von wenig Matrixmaterial kann die Oberflächenrauhigkeit weiter erhöht werden. Die Harzmenge wird so gewählt, daß sie gerade ausreicht, um die Flechtfasern mit der Pfanne zu verbinden.

## Patentansprüche

1. Implantat aus biokompatiblem Material, das auf Festigkeitseigenschaften und auf tribologische Eigenschaften optimiert ist, dadurch gekennzeichnet, daß das Implantat aus Faserverbundmaterial besteht, bei dem die Fasern gezielt zur Optimierung der Festigkeits- und tribologischen Eigenschaften ausgelegt sind.

2. Implantat nach Anspruch 1, dadurch gekennzeichnet, daß die Fasern schwerpunktmäßig auf geringen Verschleiß ausgelegt sind.

3. Implantat nach Anspruch 1 oder 2, dadurch gekennzeichnet, daß die Fasern unterschiedliche Durchmesser, insbesondere zwischen 5 und 20 µm, und/oder unterschiedliche Längen, insbesondere 50 - 500 µm haben und mit regelloser Orientierung in der Matrix eingebettet sind.

4. Implantat nach einem der vorhergehenden Ansprüche, dadurch gekennzeichnet, daß der Faseranteil im Implantatvolumen 20 - 65%, insbesondere höher als 30%, vorzugsweise 40 bis 50% ausmacht.

5. Implantat nach einem der vorhergehenden Ansprüche, dadurch gekennzeichnet, daß für einen harten Reibpartner die Fasern eine Zugfestigkeit unterhalb von 1500 MPa und einen E-Modul zwischen 150 bis 190 GPa, insbesondere 170 GPa auf Zug haben.

6. Implantat nach einem der vorhergehenden Ansprüche, dadurch gekennzeichnet, daß als Fasern Kohlenstoffasern aus Pechprecursur vorgesehen sind und daß die Matrix ein Material mit einer Druckfestigkeit oberhalb von 100 MPa ist, insbesondere ein Duroplast, z. B. Polyphenylglicidylether.

7. Implantat nach Anspruch 6, dadurch gekennzeichnet, daß das Implantat mit reinen Kohlenstoff-Fasern gefertigt ist, d.h. ohne Verwendung von Fertigungshilfsstoffen, wobei der Kohlenstoffanteil der Faser größer als 97 Gew.-%, vorzugsweise 98,6 Gew.-% beträgt, und daß der Wasserstoffanteil der Fasern unter 0,8 Gew.-%, vorzugsweise um 0,5 Gew.-% liegt, ferner daß die Fasern eine Kristallstruktur haben, die lamellenartige Schichten in radialer Richtung aufweisen.

8. Pfanne eines Gelenkimplantats nach einem der vorhergehenden Ansprüche, dadurch gekennzeichnet, daß das Material für die Pfanne (10) nach seinen tribologischen Eigenschaften gewählt wird, und daß die Pfanne (10) von einem als Anwachshilfe dienenden Flechtwerk (15) überzogen ist.

9. Pfanne nach Anspruch 8, dadurch gekennzeichnet, daß das Flechtwerk (15) in einen Zapfen (16) übergeht, der zur Drehsicherung an der Außenseite (11) der Pfanne (10) hervorragt.

10. Pfanne nach Anspruch 8 oder 9, dadurch gekennzeichnet, daß die Pfanne (10) halbkugelförmig ausgebildet ist und das Flechtwerk (15) die Stirnseite (13) zumindest teilweise umfaßt.

11. Pfanne nach Anspruch 10, dadurch gekennzeichnet, daß die Stirnseite (13) eine Ringvertiefung (14) für das Flechtwerk (15) aufweist.
